(19) Europäisches Patentamt
European Patent Office
Office européen des brevets

(11) Veröffentlichungsnummer: **0 000 368**
**B1**

(12) # EUROPÄISCHE PATENTSCHRIFT

(45) Veröffentlichungstag der Patentschrift: **29.04.81**

(51) Int. Cl.³: **C 07 J 63/00,** A 61 K 31/365, A 61 K 31/19

(21) Anmeldenummer: **78100302.5**

(22) Anmeldetag: **04.07.78**

(54) **D-Homosteroide und solche enthaltende Präparate sowie Verfahren zur Herstellung solcher D-Homosteroide.**

(30) Priorität: **06.07.77 LU 77699**

(43) Veröffentlichungstag der Anmeldung: **24.01.79 Patentblatt 79/2**

(45) Bekanntmachung des Hinweises auf die Patenterteilung: **29.04.81 Patentblatt 81/17**

(84) Benannte Vertragsstaaten: **BE CH DE FR GB LU NL SE**

(56) Entgegenhaltungen:
**DE-A-2 424 752**
**FR-A-2 244 497**
**NL-A-7 713 229**

(73) Patentinhaber: **F. HOFFMANN-LA ROCHE & CO. Aktiengesellschaft, CH-4002 Basel (CH)**

(72) Erfinder: **Fürst, Andor, Dr., Magnolienpark 14, CH-4052 Basel (CH)**
Erfinder: **Keller, Peter, Dr., Hinterlindenweg 53, CH-4153 Reinach (CH)**
Erfinder: **Müller, Marcel, Dr., Quellenweg 10, CH-4402 Frenkendorf (CH)**

(74) Vertreter: **Lederer, Franz, Dr. et al, Patentanwälte Lederer, Meyer Lucile-Grahn-Strasse 22, D-8000 München 80 (DE)**

**EP 0 000 368 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

BUNDESDRUCKEREI BERLIN

D-Homosteroide und solche enthaltende Präparate sowie Verfahren zur Herstellung solcher D-Homosteroide

Die Erfindung betrifft neue D-Homosteroide der Formel

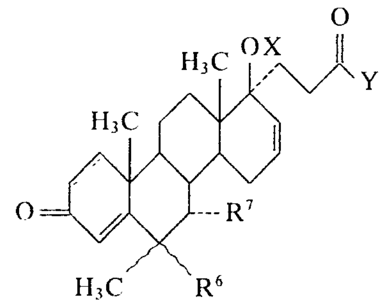

(I)

worin die punktierte Linie im A-Ring eine fakultative C – C-Bindung; X und Y zusammen eine O – C-Bindung oder X Wasserstoff und Y Hydroxy; $R^6$ Wasserstoff und $R^7$ Wasserstoff oder Acylthio oder $R^6$ und $R^7$ zusammen eine C – C-Bindung darstellen,
und physiologisch verträgliche Salze hiervon.

Die Erfindung betrifft weiterhin ein Verfahren zur Herstellung der Verbindungen I sowie pharmazeutische Präparate, welche eine Verbindung der Formel I enthalten.

Der Ausdruck »Acyl« bezieht sich auf den Rest einer gesättigten oder ungesättigten aliphatischen Carbonsäure, einer cycloaliphatischen, einer araliphatischen oder aromatischen Carbonsäure mit vorzugsweise bis zu 15 C-Atomen. Beispiele solcher Säuren sind Ameisen-, Essig-, Pivalin-, Propion-, Butter-, Capron-, Oenanth-, Undecylen-, Öl-, Cyclohexylpropion-, Cyclopentylpropion-, Phenylessig- und Benzoesäure. Besonders bevorzugte Acylgruppen sind $C_{1-7}$-Alkanoylgruppen, insbesondere Actyl.

Als physiologisch verträgliche Salze der Säuren der Formel I kommen insbesondere Alkalimetallsalze, z. B. die Na- und K-Salze sowie die Ammoniumsalze; und Erdalkalimetallsalze, z. B. die Calciumsalze in Betracht. Von den Salzen sind die Kaliumsalze bevorzugt.

Die Methylgruppe in 6-Stellung kann $\alpha$- oder $\beta$-ständig sein, bevorzugt sind die 6$\alpha$-Methyl-Isomeren.

Eine bevorzugte Gruppe von Verbindungen der Formel I sind diejenigen Verbindungen, in denen $R^7$ Acylthio, insbesondere $C_{1-7}$-Alkanoylthio ist. Weiterhin sind die Lactone der Formel I bevorzugt.

Die Verbindungen der Formel I können erfindungsgemäß dadurch hergestellt werden, daß man

a) ein D-Homosteroid der Formel

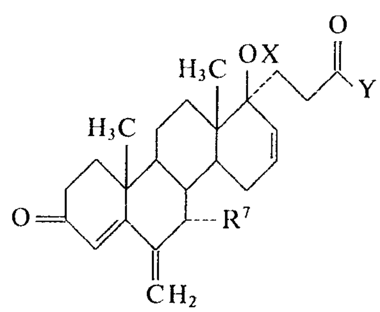

(II)

oder ein Salz davon hydriert oder isomerisiert, oder

b) ein D-Homosteroid der Formel

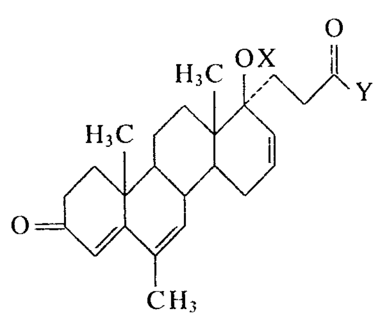

(III)

oder ein Salz davon mit einer Verbindung der Formel $R^7H$ umsetzt, oder

c) ein D-Homosteroid der Formel

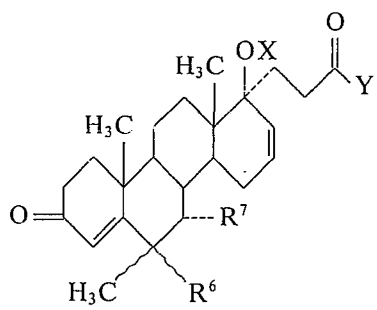

(IV)

oder ein Salz davon in 1,2-Stellung und/oder, falls $R^6$ und $R^7$ Wasserstoff sind, in 6,7-Stellung dehydriert, oder

d) in einem D-Homosteroid der Formel

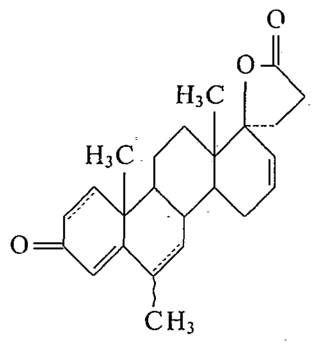

(V)

den Lactonring öffnet, oder

e) ein D-Homosteroid der Formel

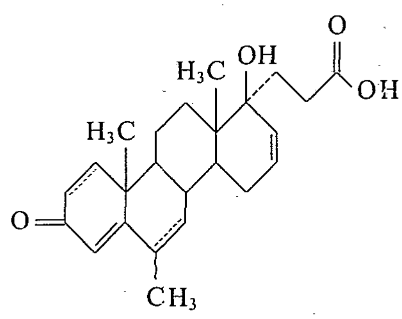

(VI)

oder ein Salz davon laktonisiert, oder

f) ein D-Homosteroid der Formel

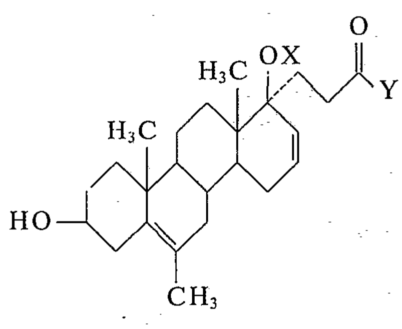

(VII)

oder ein Salz davon zu einem entsprechenden $\Delta^{4}$-, $\Delta^{4,6}$- oder $\Delta^{1,4,6}$-3-Keton oxydiert, wobei X, Y, $R^6$ und $R^7$ in den obigen Formeln die gleiche Bedeutung wie in Formel I haben und die punktierten 1,2- und 6,7-Bindungen in den Formeln V und VI fakultativ sind.

Die Hydrierung der 6-Methylengruppe in einem D-Homosteroid der Formel II gemäß Verfahrensvariante a) kann in an sich bekannter Weise mittels Hydrierkatalysatoren, z. B. Edelmetallkatalysatoren, wie Palladium, durchgeführt werden.

Die Isomerisierung der 6-Methylengruppe unter Ausbildung einer 6-Methyl-$\Delta^6$-Gruppierung kann ebenfalls in an sich bekannter Weise, z. B. katalytisch durchgeführt werden. Als Isomerisierungskatalysatoren eignen sich z. B. Metallkatalysatoren wie sie beispielsweise auch bei Hydrierungen verwendet werden, insbesondere Palladium in Äthanol. Zweckmäßig setzt man noch einen Wasserstoffdonator, wie Cyclohexen, als Aktivator für den Katalysator zu. Unerwünschte Nebenreaktionen, wie Hydrierungen durch den Wasserstoffdonator, können durch Pufferung des Reaktionsgemisches vermieden werden.

Nach der Verfahrensvariante a) werden 1,2-gesättigte Verbindungen der Formel I erhalten, in denen $R^6$ und $R^7$ Wasserstoff oder zusammen eine C—C-Bindung darstellen.

Die Einführung eines Acylthio-Substituenten $R^7$ in ein D-Homosteroid der Formel III (Verfahrensvariante b) kann in an sich bekannter Weise durch Behandlung des Steroids mit einer entsprechenden Thiocarbonsäure erfolgen. Die Reaktion kann in einem inerten Lösungsmittel wie einem Äther, z. B. Dioxan oder Tetrahydrofuran, oder einem Alkohol, wie Methanol oder Äthanol, oder in einem Chlorkohlenwasserstoff, wie Chloroform durchgeführt werden; das Reagens, z. B. die Thiocarbonsäure wird zweckmäßigerweise im Überschuß eingesetzt und kann dabei als Lösungsmittel dienen.

Mittels der Verfahrensvariante b) erhält man 1,2-gesättigte D-Homosteroide der Formel I, in denen $R^6$ Wasserstoff und $R^7$ Acylthio darstellen.

Die 1,2-Dehydrierung eines D-Homosteroids der Formel IV (Verfahrensvariante c) kann in an sich bekannter Weise z. B. auf mikrobiologischem Wege oder mittels Dehydrierungsmitteln wie Selendioxyd, 2,3-Dichlor-5,6-dicyanobenzochinon, Chloranil, Thalliumtriacetat oder Bleitetraacetat vorgenommen werden. Geeignete Mikroorganismen für die 1,2-Dehydrierung sind beispielsweise Schizomyceten, insbesondere solche der Genera Arthrobacter, z. B. A. simplex ATCC 6946; Bacillus, z. B. B. lentus ATCC 13805 und B. sphaericus ATCC 7055; Pseudomonas, z. B. P. aeruginosa IFO 3505; Flavobacterium, z. B. F. flavescens IFO 3058; Lactobacillus, z. B. L. brevis IFO 3345 und Nocardia, z. B. N. opaca ATCC 4276.

Die Einführung einer $\Delta^6$-Doppelbindung kann z. B. mit einem substituierten Benzochinon, wie Chloranil [vgl. J. Am. Chem. Soc. 82, 4293 (1960); 81, 5951 (1959)] oder mit 2,3-Dichlor-5,6-dicyanobenzochinon oder mit Mangandioxid [vgl. J. Am. Chem. Soc. 75, 5932 (1953)] erfolgen.

Mit 2,3-Dichlor-5,6-dicyanobenzochinon oder Chloranil kann auch direkt die 1,4,6-Trisdehydro-Verbindung erhalten werden.

Die Verfahrensvariante c) führt zu 4,6-Dien-, 1,4-Dien- und 1,4,6-Trien-D-Homosteroiden der Formel I.

Die Aufspaltung des Lactonrings gemäß Verfahrensvariante d) kann in an sich bekannter Weise durchgeführt werden, z. B. mittels einer Base, wie Kalium- oder Natriumhydroxyd, in einem Lösungsmittel, z. B. einem Alkohol wie Methanol, Äthanol oder Isopropanol, bei einer Temperatur zwischen etwa 0° C und Rückflußtemperatur des Reaktionsgemisches, zweckmäßig bei etwa 50° C. Die so erhaltenen, der verwendeten Base entsprechenden Salze können durch Ansäuern, z. B. mittels Chlorwasserstoffsäure, in die freien Säuren übergeführt werden. Letztere können durch Umsetzung mit geeigneten Basen in Salze übergeführt werden.

Die Verfahrensvariante d) liefert Verbindungen der Formel I, in denen X Wasserstoff und Y Hydroxy darstellen, und deren Salze.

Die Lactonisierung einer Verbindung der Formel VI (Variante e) oder eines Salzes hiervon kann in an sich bekannter Weise durchgeführt werden, z. B. mittels einer starken Säure, wie Chlorwasserstoffsäure, Schwefelsäure oder p-Toluolsulfonsäure, in einem Lösungsmittel, z. B. Wasser, einem Alkohol, wie Methanol, oder Gemischen hiervon, bei einer Temperatur zwischen etwa −50° und 100° C, zweckmäßig bei Zimmertemperatur.

Die Oxydation einer Verbindung der Formel VII (Verfahrensvariante f) kann nach Oppenauer, z. B. mittels Aluminiumisopropylat, oder mit Oxydationsmitteln wie Chromtrioxid (z. B. Jones'Reagens), oder nach Pfitzner-Moffatt mittels Dimethylsulfoxid/Dicyclohexylcarbodiimid (wobei das primär erhaltene $\Delta^5$-3-Keton anschließend zum $\Delta^4$-3-Keton isomerisiert werden muß), oder mittels Pyridin/$SO_3$ vorgenommen werden. Bei Anwendung der vorstehend genannten Oxydationsmittel wie $Br_2$/LiBr/$Li_2CO_3$ in Dimethylformamid oder bei der Oxydation nach Oppenauer in Gegenwart von Benzochinon liefert die Oxydation eine 3-Keto-$\Delta^{4,6}$-Gruppierung. Füpr die Oxydation zu einem 3-Keto-$\Delta^{1,4,6}$-steroid ist beispielsweise 2,3-Dichlor-5,6-dicyano-benzochinon (DDQ) geeignet.

Die Ausgangsverbindungen der Formeln II-VII können, sofern ihre Herstellung nicht bereits bekannt oder nachstehend beschrieben ist, in Analogie zu bekannten bzw. den nachstehend beschriebenen Methoden hergestellt werden.

Die D-Homosteroide der Formel I und die Salze hiervon zeigen pharmakologische Wirkung. Unter anderem sind sie diuretisch wirksam und geeignet, die Wirkung von Aldosteron oder von Desoxycorticosteron-acetat zu blockieren und können somit beispielsweise als kaliumsparende Diuretika oder zur Ausschwemmung von Ödemen Anwendung finden. Als Dosierungsrichtlinie kommen etwa 0,1 bis 10 mg/kg pro Tag in Betracht.

Diskussion zum Stand der Technik

Im Gegensatz zu den erfindungsgemäßen D-Homosteroiden der Formel I enthalten die aus der französischen Anmeldung Nr. 2 244 497 und der deutschen Offenlegungsschrift Nr. 2 424 752 bekannten D-Homosteroide keinen Methylrest in 6-Stellung Überraschender Weise sind die erfindungsgemäßen 6-Methyl-D-homosteroide den vorbekannten 6-unsubstituierten D-Homosteroiden beträchtlich überlegen. Wie beispielsweise aus dem nachstehenden Versuchsbericht ersichtlich, haben die erfindungsgemäßen D-Homosteroide der Formel I (Verbindungen A und B im Versuchsbericht) eine stärkere kaliumsparende diuretische Wirksamkeit als die aus der deutschen Offenlegungsschrift Nr. 2 424 752 bekannten D-Homosteroide (Verbindungen C und D).

Versuchsbericht

Die Testsubstanz wird episiotomisierten und catheterisierten Hunden in einer Gelatinekapsel peroral verabreicht. UnmittelbardanachwerdendenTieren 0,5 µg/kg Aldosteron subcutan injiziert. Der Harn der Tiere wird über eine Periode von 6 Stunden gesammelt. Das aus der Gesamtausscheidung errechnete $Na^+/K^+$ Verhältnis wird als Maß für den Aldosteron-Antagonismus gewonnen. Bei Durchführung des Versuchs mit 7α-Acetylthio-6α-methyl-3-oxo-D-homo-17aα-pregna-4,16-dien-21,17a-carbolacton (Verbindung A), 7α-Acetylthio-6α-methyl-3-oxo-D-homo-17aα-pregna-1,4,16-trien-21,17a-carbolacton (Verbindung B), 7α-Acetylthio-3-oxo-D-homo-17aα-pregna-4-en-21,17a-carbolacton (Verbindung C) und 7α-Acetylthio-3-oxo-D-homo-17aα-pregna-4,16-dien-21,17a-carbolacton (Verbindung D) wurden folgende Ergebnisse erhalten:

| Verbindung | Dosis mg/kg | $Na^+/K^+$ Verhältnis |
|---|---|---|
| A | 4 | 1,34 |
| B | 4 | 2,55 |
| C | 4 | 0,58 |
| D | 4 | 0,81 |
| Kontrolle | 0 | 0,64 |

Die Verbindungen der Formel I und die Salze hiervon können als Heilmittel z. B. in Form pharmazeutischer Präparate Verwendung finden, welche sie oder ihre Salze in Mischung mit einem für die enterale oder parenterale Applikation geeigneten organischen oder anorganischen inerten Trägermaterial, wie z. B. Wasser, Gelatine, Gummi arabicum, Milchzucker, Stärke, Magnesiumstearat, Talk, pflanzliche Öle, Polyalkylenglykole, Vaseline, usw. enthalten. Die pharmazeutischen Präparate können in fester Form, z. B. als Tabletten, Dragées, Suppositorien, Kapseln; oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die Herstellung der Arzneimittel kann in an sich bekannter Weise erfolgen, indem man die Verbindung der Formel I mit zur therapeutischen Verabreichung geeigneten, nicht-toxischen, inerten, an sich in solchen Präparaten üblichen festen und/oder flüssigen Trägermaterialien, wie z. B. den vorstehend genannten, vermischt und gegebenenfalls in die gewünschte Form bringt.

Beispiel 1

Eine Mischung von 1,0 g 6-Methylen-3-oxo-D-homo-17aα-pregna-4,16-dien-21,17a-carbolacton, 0,5 g Natriumacetat, 50 mg 5% Palladium/Kohle und 35 ml Äthanol wurde 15 Stunden unter Rückfluß erhitzt. Gleichzeitig wurden pro Stunde 2 ml einer 0,5%igen Lösung von Cyclohexen in Äthanol zugetropft. Zur Aufarbeitung wurde der Katalysator abfiltriert und das Filtrat im Vakuum eingedampft. Der Rückstand wurde auf 55 g Silicagel chromatographiert. Mit Methylenchlorid-Aceton (98 : 2) konnten 820 mg reines 6-Methyl-3-oxo-D-homo-17aα-pregna-4,6,16-trien-21,17a-carbolacton eluiert werden. Smp. 197 – 198° (aus Aceton-Hexan). $[\alpha]_D^{25} = +41°$ (c = 0,1 in Dioxan), $\varepsilon_{287} = 22\,100$.

Diese Verbindung kann auch durch Ansäuern einer Lösung des Kalium-17a-hydroxy-6-methyl-3-oxo-D-homo-17aα-pregna-4,6,16-trien-21-carboxylats mit verdünnter Salzsäure erhalten werden.

Das Ausgangsmaterial wurde wie folgt hergestellt:

3-Oxo-D-homo-17aα-pregna-4,16-dien-21,17a-carbolacton wurde mit Pyrrolidin in Methanol in das Enamin 3-(1-Pyrrolidinyl)-D-homo-17aα-prgna-3,5-16-trien-21,17a-carbolacton übergeführt. Durch Reaktion dieser Verbindung mit Formaldehyd in Benzol-Methanol-Wasser erhielt man 6β-Hydroxymethyl-3-oxo-D-homo-17aα-pregna-4,16-dien-21,17a-carbolacton, Smp. 246-249°, $[\alpha]_D^{25} = +6°$ (c=0,1 in Dioxan). Behandlung dieser Verbindung mit wässeriger HCl in Dioxan führte zu 6-Methylen-3-oxo-D-homo-17aα-pregna-4,16-dien-21,17a-carbolacton, Smp. 216-220°, $[\alpha]_D^{25} = +175°$ (c=0,1 in Dioxan).

## Beispiel 2

Eine Lösung von 1,0 g 6-Methyl-3-oxo-D-homo-17aα-pregna-4,6,16-trien-21,17a-carbolacton in 10 ml Thioessigsäure wurde 6 Stunden zum Rückfluß erhitzt. Die überschüssige Thioessigsäure wurde im Vakuum abgedampft und der Rückstand auf 100 g Silicagel chromatographiert. Mit Hexan-Aceton konnten 0,9 g reines 7α-Acetylthio-6α-methyl-3-oxo-D-homo-17aα-pregna-4,16-dien-21,17a-carbolacton eluiert werden. Smp. 215–217° (aus Aceton-Hexan). $[\alpha]_D^{25} = -16°$ (c=0,1 in Dioxan) · $\varepsilon_{237} = 19\,900$.

## Beispiel 3

Eine Lösung von 1,0 g 7α-Acetylthio-6α-methyl-3-oxo-D-homo-17aα-pregna-4,16-dien-21,17a-carbolacton und 0,9 g DDQ in 50 ml Dioxan wurde 48 Stunden unter Rückfluß gekocht. Die abgekühlte Lösung wurde durch 20 g Alox Akt. II filtriert und das Produkt mit 300 ml Essigester vollständig eluiert. Das Eluat ergab nach Abdampfen des Lösungsmittels 1,0 g Rohprodukt, das auf 50 g Silicagel chromatographiert wurde. Mit Hexan-Aceton (6 : 1) konnten 730 mg reines 7α-Acetylthio-6α-methyl-3-oxo-D-homo-17aα-pregna-1,4,16-trien-21,17a-carbolacton eluiert werden. Smp. 160–162°. $[\alpha]_D^{25} = -29°$ (c=1,0 in Dioxan) · $\varepsilon_{240} = 17\,400$.

## Beispiel 4

Zu einer Lösung von 700 mg 6-Methyl-3-oxo-D-homo-17aα-pregna-4,6,16-trien-21,17a-carbolacton in 7 ml 2-Propanol gab man eine Lösung von 126 mg KOH (85%) in 0,68 ml Wasser und erhitzte die Mischung 30 Minuten zum Rückfluß. Die Lösung wurde im Vakuum zur Trockene verdampft, der Rückstand durch Zugabe und Abdampfen von abs. Alkohol wasserfrei gemacht. Den Rückstand suspendiert man in 30 ml Essigester und nutschte das Produkt ab. Nach Trocknen über Nacht im Vakuum bei 60° erhielt man 800 mg reines 17a-Hydroxy-6-methyl-3-oxo-D-homo-17aα-pregna-4,6,16-trien-21-carbonsäure-Kaliumsalz. $[\alpha]_D^{25} = -120°$ (c=0,1 in Methanol) · $\varepsilon_{290} = 22\,800$.

## Beispiel 5

Eine Mischung von 1,0 g 6-Methylen-3-oxo-D-homo-17aα-pregna-4,16-dien-21,17a-carbolacton, 200 mg 5% Pd/C, 5 ml Benzol und 5 ml Cyclohexen wurde 12 Stunden unter Argon am Rückfluß erhitzt. Die abgekühlte Mischung wurde filtriert und das Filtrat im Vakuum eingedampft. Durch Umkristallisieren des Rohproduktes aus Alkohol und Aceton erhielt man reines 6β-Methyl-3-oxo-D-homo-17aα-pregna-4,16-dien-21,17a-carbolacton, Smp. 238–240°. $[\alpha]_D^{25} = +2°$ (c=0,1 in Dioxan) · $\varepsilon_{241} = 15\,700$.

## Beispiel 6

2,5 g 3β-Hydroxy-6-methyl-D-homo-17aα-pregna-5,16-dien-21,17a-carbolacton werden in 67 ml Dimethylformamid gelöst. Dem Gemisch werden 3,1 g Lithiumbromid und 3,1 g Lithiumcarbonat zugegeben. Die weiße Suspension wird unter Rühren und Argonbegasung auf 80°C erwärmt. Bei dieser Temperatur wird innert 80 Minuten eine Lösung von 2,62 g Brom in 21 ml Dioxan zugetropft. Nach beendigter Zugabe wird die gelb-orange Suspension noch weitere 30 Minuten bei 80°C gerührt. Zur Aufarbeitung wird die erhaltene Lösung mit 6 ml Eisessig versetzt, auf 1000 ml Wasser gegossen und mit 3 x 400 ml Äther-Methylenchlorid (4 : 1) extrahiert. Die organischen Phasen werden mit ges. Natriumchloridlösung neutral gewaschen, mit Magnesiumsulfat getrocknet und zur Trockene eingeengt. Das Rohprodukt wird durch Chromatographie an der 100fachen Menge Kieselgel mit Methylenchlorid-Aceton (98 : 2) gereinigt. Die das Produkt enthaltenden Fraktionen werden zusammengenommen und aus Aceton-Hexan umkristallisiert. Man erhält so 3-Oxo-6-methyl-D-homo-

17a$\alpha$-pregna-4,6,16-trien-21,17a-carbolacton in Form von farblosen Kristallen vom Schmelzpunkt 197–198°, $[\alpha]_D$ = +41° (Dioxan c=0,1).

Das Ausgangsmaterial kann wie folgt hergestellt werden: Dimethylsulfoxoniummethylid wird mit 3$\beta$-Hydroxy-6-methyl-androst-5-en-17-on in Dimethylsulfoxid zu 17,20-Epoxy-3$\beta$-hydroxy-6-methyl-21-norpregn-5-en umgesetzt. Letzteres wird mit Ammoniak unter Druck zu 20-Amino-3$\beta$,17-dihydroxy-6-methyl-21-norpregn-5-en geöffnet. Durch Demjanov-Ringerweiterung wird 3$\beta$-Hydroxy-6-methyl-D-homoandrost-5-en-17a-on erhalten, welches durch Bromierung mit Kupfer II-bromid in siedendem Methanol und nachfolgende Bromwasserstoffabspaltung mit Calciumcarbonat in siedendem Dimethylacetamid über 17$\alpha$-Brom-3$\beta$-hydroxy-6-methyl-D-homoandrost-5-en-17a-on in 3$\beta$-Hydroxy-6-methyl-D-homoandrosta-5,16-dien-17a-on übergeführt wird. Eine Lithium-Grignard-Reaktion mit 3-Brompropionaldehyd-dimethylacetal und nachfolgende Acetylierung mit Acetanhydrid-Pyridin liefern 3$\beta$-Acetoxy-21-dimethylacetal-17a-hydroxy-6-methyl-D-homo-17a$\alpha$-pregna-5,16-dien. Dieses Acetal wird mit einer wäßrigen 70%igen Essigsäurelösung zum entsprechenden Aldehyd gespalten. Dieser cyclisiert spontan zu 3$\beta$-Acetoxy-6-methyl-D-homo-17a$\alpha$-pregna-5,16-dien-21, 17a-carbolactol. Jones-Oxidation liefert das entsprechende 21,17a-carbolacton, das mit Kaliumcarbonat in Methanol zu 3$\beta$-Hydroxy-6-methyl-D-homo-17a$\alpha$-pregna-5,16-dien-21,17a-carbolacton verseift wird.

Beispiel A

Eine Tablette zur oralen Verabreichung kann folgende Zusammensetzung aufweisen:

| | |
|---|---|
| Verbindung der Formel I oder Salz hiervon | 25 mg |
| Maisstärke | 100 mg |
| Lactose | 50 mg |
| Polyvinylpyrrolidon | 15 mg |
| Magnesiumstearat | 2 mg |

Beispiel B

Eine Kapsel zur oralen Verabreichung kann folgende Zusammensetzung aufweisen:

| | |
|---|---|
| Verbindung der Formel I oder Salz hiervon | 25 mg |
| Maisstärke | 125 mg |
| Lactose | 125 mg |

**Patentansprüche**

1. D-Homosteroide der Formel

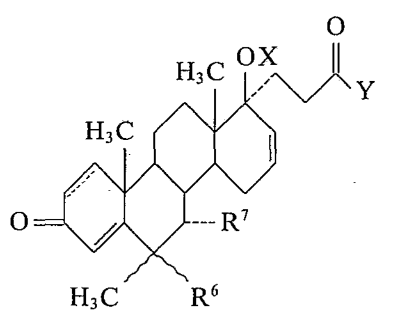

(I)

worin die punktierte Linie im A-Ring fakultative C–C-Bindung; X und Y zusammen eine O–C-Bindung oder X Wasserstoff und Y Hydroxy; $R^6$ Wasserstoff und $R^7$ Wasserstoff oder Acylthio oder $R^6$ und $R^7$ zusammen eine C–C-Bindung darstellen,
und physiologisch verträgliche Salze hiervon.

2. D-Homosteroide und Salze davon gemäß Anspruch 1, wobei $R^7$ Acylthio ist.

3. D-Homosteroid-lactone gemäß Anspruch 1 oder 2.

4. D-Homosteroide und Salze davon gemäß Anspruch 1, 2 oder 3, wobei $R^7$ $C_{1-7}$-Alkanoylthio ist.

5. 7$\alpha$-Acetylthio-6$\alpha$-methyl-3-oxo-D-homo-17a$\alpha$-pregna-1,4,16-trien-21,17a-carbolacton gemäß Anspruch 1.

6. Pharmazeutische Präparate, gekennzeichnet durch einen Gehalt an einem D-Homosteroid der Formel I gemäß Definition in Anspruch 1 oder einem physiologisch verträglichen Salz davon.

7. Verfahren zur Herstellung von D-Homosteroiden der Formel

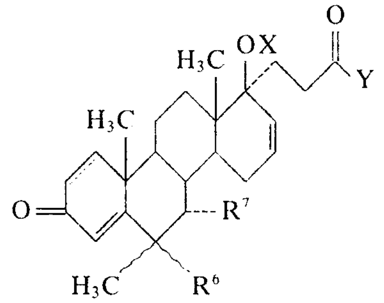

(I)

worin die punktierte Linie im A-Ring fakultative C – C-Bindung; X und Y zusammen eine O – C-Bindung oder X Wasserstoff und Y Hydroxy; $R^6$ Wasserstoff und $R^7$ Wasserstoff oder Acylthio oder $R^6$ und $R^7$ zusammen eine C – C-Bindung darstellen,
und physiologisch verträgliche Salze hiervon, dadurch gekennzeichnet daß man

a) ein D-Homosteroid der Formel

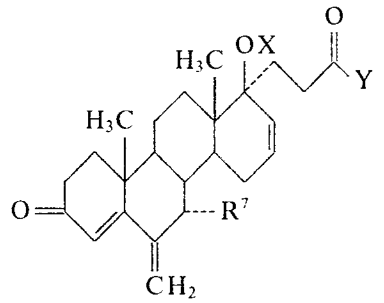

(II)

oder ein Salz davon hydriert oder isomerisiert, oder

b) ein D-Homosteroid der Formel

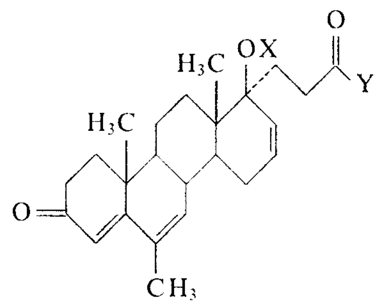

(III)

oder ein Salz davon mit einer Verbindung der Formel $R^7H$ umsetzt, oder

c) ein D-Homosteroid der Formel

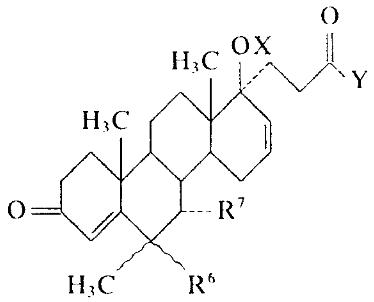

(IV)

oder ein Salz davon in 1,2-Stellung und/oder, falls $R^6$ und $R^7$ Wasserstoff sind, in 6,7-Stellung dehydriert, oder

d) in einem D-Homosteroid der Formel

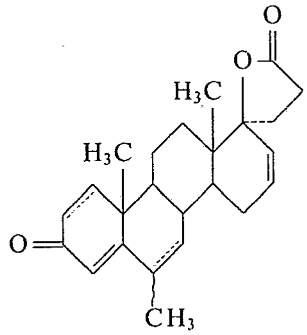

(V)

den Lactonring öffnet, oder

e) ein D-Homosteroid der Formel

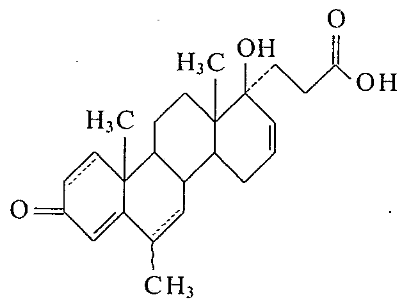

(VI)

oder ein Salz davon laktonisiert, oder

f) ein D-Homosteroid der Formel

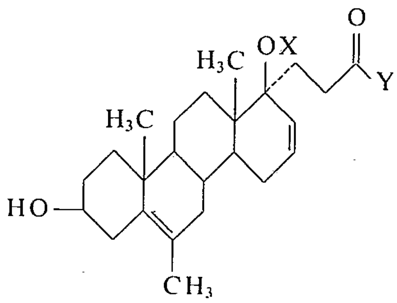

(VII)

oder ein Salz davon zu einem entsprechenden $\Delta^4$-, $\Delta^{4,6}$- oder $\Delta^{1,4,6}$-3-Keton oxydiert, wobei X, Y, $R^6$ und $R^7$ in den obigen Formeln die gleiche Bedeutung wie in Formel I haben und die punktierten 1,2- und 6,7-Bindungen in den Formeln V und VI fakultativ sind.

8. Verfahren nach Anspruch 7, dadurch gekennzeichnet, daß man D-Homosteroide der Formel I oder Salze davon herstellt, in denen $R^7$ Acylthio darstellt.

9. Verfahren nach Anspruch 7 oder 8, dadurch gekennzeichnet, daß man D-Homosteroid-lactone der Formel I herstellt.

10. Verfahren nach Anspruch 7, 8 oder 9, dadurch gekennzeichnet, daß man D-Homosteroide der Formel I herstellt, in denen $R^7$ $C_{1-7}$-Alkanoylthio ist.

11. Verfahren nach einem der Ansprüche 7—10, dadurch gekennzeichnet, daß man 7α-Acetylthio-6α-methyl-3-oxo-D-homo-17aα-pregna-1,4,16-trien-21,17a-carbolacton herstellt.

**Claims**

1. D-homosteroids of the formula

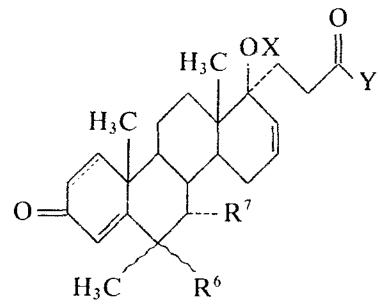

(I)

wherein the dotted line in the A-ring represents an optional carbon-carbon bond, X and Y together represent an oxygen-carbon bond or X represents a hydrogen atom and Y represents a hydroxy group, and $R^6$ represents a hydrogen atom and $R^7$ represents a hydrogen atom or an acylthio group or $R^6$ and $R^7$ together represent a carbon-carbon bond,
and physiologically compatible salts thereof.

2. D-homosteroids and salts thereof according to claim 1, wherein $R^7$ represents an acylthio group.

3. D-Homosteroid-lactones according to claim 1 or claim 2.

4. D-homosteroids and salts thereof according to claim 1, 2 or 3, wherein $R^7$ represents a $C_{1-7}$-alkanoylthio group.

5. 7α-Acetylthic-6α-methyl-3-oxo-D-homo-17aα-pregna-1,4,16-triene-21,17a-carbolactone according to claim 1.

6. Pharmaceutical preparations containing a D-homosteroid of formula I as set forth in claim 1 or physiologically compatible salts thereof.

7. A process for the manufactur of D-homosteroids of the formula

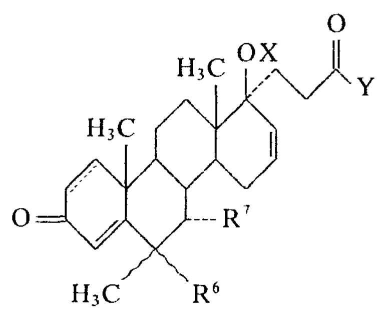

(I)

wherin the dotted line in the A-ring represents an optional carbon-carbon bond, X and Y together represent an oxygen-carbon bond or X represents a hydrogen atom and Y represents a hydroxy group, and $R^6$ represents a hydrogen atom and $R^7$ represents a hydrogen atom or an acylthio group or $R^6$ and $R^7$ together represent a carbon-carbon bond,
and physiologically compatible salts thereof, which process comprises

a) hydrogenating or isomerising a D-homosteroid of the formula

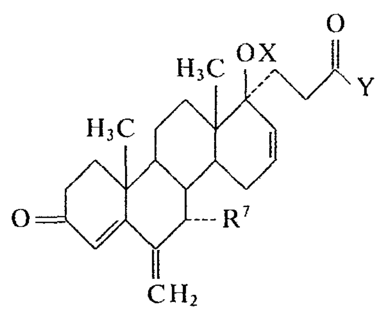

(II)

or a salt thereof, or

b) reacting a D-homosteroid of the formula

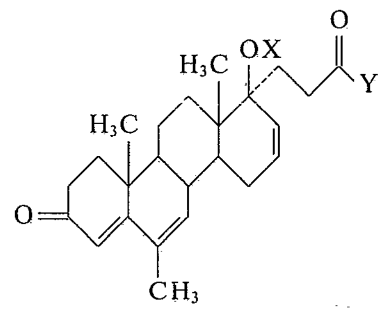

(III)

or a salt thereof with a compound of the formula $R^7H$, or

c) dehydrogenating a D-homosteroid of the formula

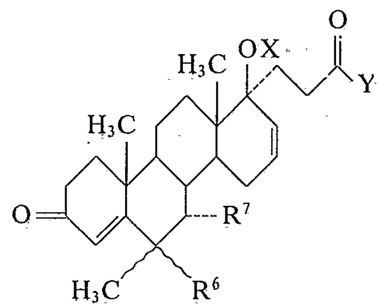

(IV)

or a salt thereof in the 1,2-position and/or, where $R^6$ and $R^7$ each represent a hydrogen atom, in the 6,7-position, or

d) cleaving the lactone ring in a D-homosteroid of the formula

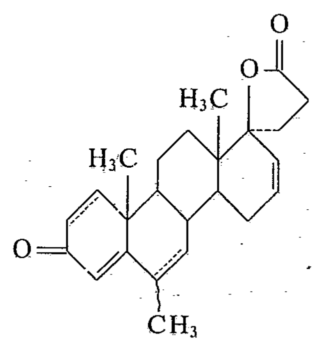

(V)

or

e) lactonising a D-homosteroid of the formula

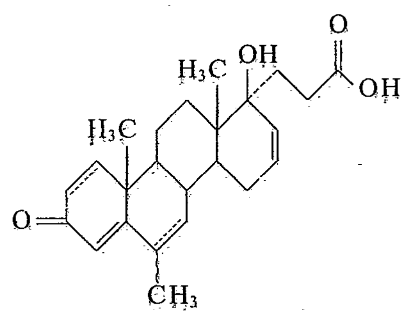

(VI)

or a salt thereof, or

f) oxidising a D-homosteroid of the formula

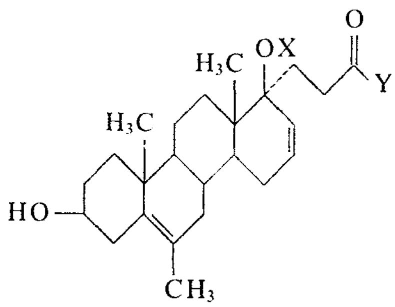

(VII)

or a salt thereof to give a corresponding $\Delta^4$-, $\Delta^{4,6}$- or $\Delta^{1,4,6}$-3-ketone, X, Y, $R^6$ and $R^7$ in the above formulae having the same significance as in formula I and the dotted bonds 1,2 and 6,7 in formulae V and VI are optional.

8. A process according to claim 7, characterized in that a D-homosteroid of formula I, or a salt thereof, in which $R^7$ represents an acylthio group, is manufactured.

9. A process according to claim 7 or claim 8, characterized in that a D-homosteroid-lactone of formula I is manufactured.

10. A process according to claim 7, 8 or 9, characterized in that D-homosteroids fo formula I, in which $R^7$ represents a $C_{1-7}$-alkanoylthio group, are manufactured.

11. A process according to any one of claims 7 to 10 inclusive, characterized in that 7α-acetylthio-6α-methyl-3-oxo-D-homo-17aα-pregna-1,4,16-triene-21,17a-carbolactone is manufactured.

**Revendications**

1. D-homostéroïdes de formule

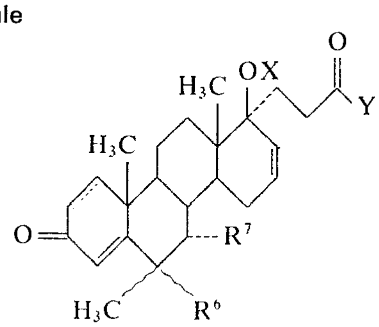

(I)

où la ligne pointillée dans le noyau A représente une liaison C–C facultative; X et Y représentent ensemble une liaison O–C, ou X un hydrogène et Y un hydroxy; $R^6$ un hydrogène et $R^7$ un hydrogène ou un acylthio, ou $R^6$ et $R^7$, ensemble, une liaison C–C,
et leurs sels physiologiquement acceptables.

2. D-homostéroïdes et leurs sels selon la revendication 1, où $R^7$ est un acylthio.

3. Lactones D-homostéroïdiques selon la revendication 1 ou 2.

4. D-homostéroïdes et leurs sels selon la revendication 1, 2 ou 3, où $R^7$ est un alcanoylthio en $C_1$ à $C_7$.

5. 7α-acétylthio-6α-méthyl-3-oxo-D-homo-17aα-prégna-1,4,16-triène-21,17a-carbolactone selon la revendication 1.

6. Préparations pharmaceutiques, caractérisées en ce qu'elles contiennent un D-homostéroïde de formule I selon la définition de la revendication 1 ou un de ses sels physiologiquement acceptables.

7. Procédé de préparation de D-homostéroïdes de formule

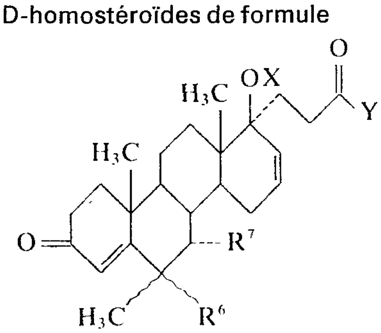

(I)

où la ligne pointillée dans le noyau A représente une liaison C–C facultative; X et Y, ensemble, une

liaison O – C ou X un hydrogène et Y un hydroxy; $R^6$ un hydrogène et $R^7$ un hydrogène ou un acylthio ou $R^6$ et $R^7$, ensemble, une liaison C – C, et leurs sels physiologiquement acceptables, caractérosé en ce que

a) On hydrogène ou on isomérise un D-homostéroïde de formule

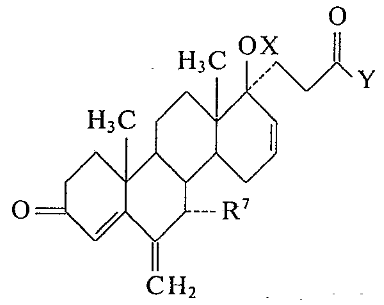

(II)

ou un de ses sels, ou

b) On fait réagir un D-homostéroïde de formule

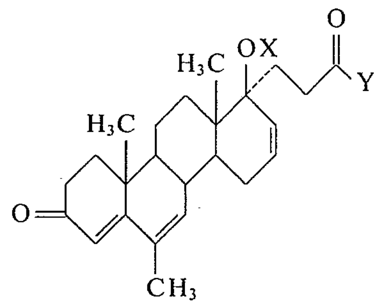

(III)

ou un de ses sels avec un composé de formule $R^7H$, ou

c) On déshydrogène un D-homostéroïde de formule

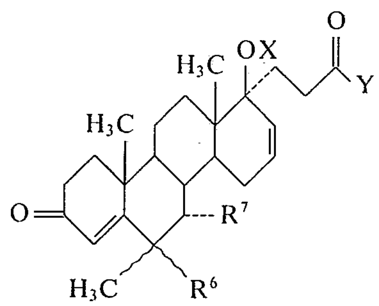

(IV)

ou un de ses sels en position 1,2 et/ou, si $R^6$ et $R^7$ sont des hydrogènes, en position 6,7, ou

d) on ouvre le noyau lactone dans un D-homostéroïde de formule

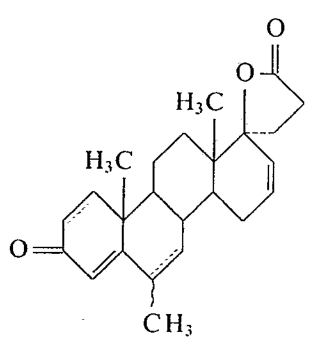

(V)

ou

e) on lactonise un D-homostéroïde de formule

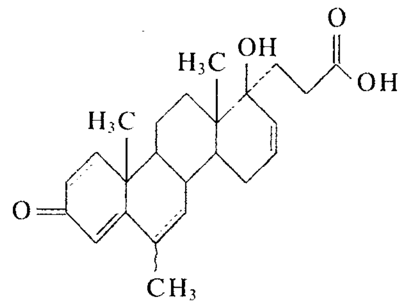

(VI)

ou un de ses sels, ou

f) on oxyde un D-homostéroïde de formule

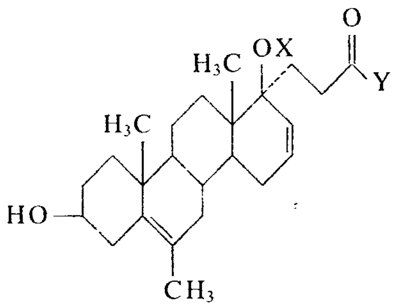

(VII)

ou un de ses sels en une $\Delta^{4}$-, $\Delta^{4,6}$- ou $\Delta^{1,4,6}$-3-cétone, où X, Y, $R^6$ et $R^7$ dans les formules ci-dessus ont la même signification que dans la formule I et où les liaisons pointillées 1,2 et 6,7 dans les formules V et VI sont facultatives.

8. Procédé selon la revendication 7, caractérisé en ce qu'on prépare des D-homostéroïdes de formule I ou leurs sels, où $R^7$ représente un acylthio.

9. Procédé selon la revendication 7 ou 8, caractérisé en ce qu'on préparè des lactones D-homostéroïdiques de formule I.

10. Procédé selon la revendication 7, 8 ou 9, caractérisé en ce qu'on prépare des D-hmostéroïdes de formule I, où $R^7$ est un alcanoylthio en $C_1$ à $C_7$.

11. Procédé selon l'une des revendications 7 à 10, caractérisé en ce qu'on prépare la 7α-acétylthio-5α-méthyl-3-oxo-D-homo-17aα-pr+egna-1,4,16-triène-21, 17a-carbolactone.